Europäisches Patentamt

**European Patent Office**

**Office européen des brevets**

(19)

(11) Veröffentlichungsnummer: **0 175 228**
**A2**

(12) # EUROPÄISCHE PATENTANMELDUNG

(21) Anmeldenummer: **85111254.0**

(22) Anmeldetag: **06.09.85**

(51) Int. Cl.⁴: **C 07 C 85/24**

(30) Priorität: **15.09.84 DE 3433982**

(43) Veröffentlichungstag der Anmeldung: **26.03.86**
**Patentblatt 86/13**

(84) Benannte Vertragsstaaten: **BE CH DE FR GB IT LI NL**

(71) Anmelder: **HOECHST AKTIENGESELLSCHAFT,**
**Postfach 80 03 20, D-6230 Frankfurt am Main 80 (DE)**

(72) Erfinder: **Eichler, Klaus, Dr., Im Traminer 10,**
**D-6236 Eschborn 2 (DE)**
Erfinder: **Arpe, Hans-Jürgen, Prof. Dr.,**
**de-Ridder-Weg 10, D-6230 Frankfurt am Main 80 (DE)**
Erfinder: **Leupold, Ernst Ingo, Dr., Am Zäunefeld 15,**
**D-6392 Neu-Anspach (DE)**

(54) **Verfahren zur Isomerisierung von Alkylanilinen.**

(57)    Die Erfindung betrifft ein Verfahren zur Isomerisierung von Alkylanilinen, deren Alkylgruppe 2 bis 6 C-Atome hat. Die Isomerisierung wird an Zeolith-Katalysatoren durchgeführt. Besonders geeignet sind synthetische Zeolithe vom Pentasil-, Mordenit- oder Faujasit-Typ in ihrer sauren Form. Insbesondere ist Gegenstand der Erfindung ein Verfahren zur Isomerisierung von Ethylanilinen an einem Zeolith.

EP 0 175 228 A2

- 1 -

HOECHST AKTIENGESELLSCHAFT    HOE 84/F 217        Dr.MA/mk

Verfahren zur Isomerisierung von Alkylanilinen

Die vorliegende Erfindung betrifft ein Verfahren zur Isomerisierung von Alkylanilinen, deren Alkylgruppe zwei bis
sechs C-Atome hat.

2-Alkylaniline sind durch Alkylierung von Anilin mit Ethylen darstellbar (US-PS 3 923 892, US-PS 3 862 233, US-PS
3 649 693).

2-Alkylaniline lassen sich auch durch Reduktion der betreffenden Nitroalkylbenzole herstellen, ebenso 4-Alkylaniline.
Für 3-Alkylaniline wird diese Methode dagegen kaum angewandt, da die 3-Nitroalkylbenzole nicht durch Nitrierung
von Alkylbenzol erhältlich sind.

Zur Herstellung von 3-Alkyaniline müssen daher andere
Wege beschritten werden, so wurde zum Beispiel zur Herstellung von 3-Ethylanilin die Reduktion von 3-Nitroacetophenon
vorgeschlagen (Chemical Abstracts 85, 108 399 f). Einfache
und preiswerte Methoden zur Herstellung von 3-Alkylanilinen
wurden jedoch bisher noch nicht beschrieben.

Dagegen ist bereits bekannt, daß sich o-, m- oder p-Toluidin an einem Zeolith-Katalysator isomerisieren lassen
(EP-A1-92103).

Gegenstand der Erfindung ist ein Verfahren zur Isomerisierung von Alkylanilinen, deren Alkylgruppe zwei bis sechs C-
Atome hat, dadurch gekennzeichnet, daß die Isomerisierung
an einem Zeolith-Katalysator durchgeführt wird. Die Alkylgruppe kann geradkettig oder verzweigt sein. Insbesondere
ist Gegenstand der Erfindung ein Verfahren zur Isomerisierung von Ethylanilin an einem Zeolith-Katalysator.

Aufgrund des Standes der Technik war es überraschend, daß sich Alkylaniline derart gut, wie die Beispiele zeigen, isomerisieren lassen. Es war eine Abspaltung von Olefinen unter Bildung von Anilin in erheblichem Ausmaß zu erwarten, im Unterschied zur Isomerisierung des Toluidins, wo dies nicht möglich ist. Eine solche Olefinabspaltung tritt jedoch nur in geringem Maß ein.

Beispiel 1 zeigt, daß das flüssige Produktgemisch bei der Isomerisierung von 2-Ethylanilin bis zu 40 % 3-Ethylanilin enthält; bei der Isomerisierung von 4-Ethylanilin sind es sogar bis zu 60 %. Ein Nachlassen der Aktivität des Katalysators ist während eines Gesamtdauer von 37 Stunden nicht zu beobachten. Das erfindungsgemäße Verfahren ermöglicht demnach die Herstellung von 3-Alkylanilinen auf einfache Weise und mit hoher Ausbeute.

Zur Durchführung des erfindungsgemäßen Verfahrens wird ein Alkylanilin oder ein Gemisch aus Alkylanilinen mit dem Zeolith-Katalysator in flüssiger oder gasförmiger Phase in Kontakt gebracht.

Als Zeolithe eignen sich im allgemeinen sowohl natürliche wie auch synthetische Zeolithe, vorzugsweise synthetische Zeolithe vom Pentasil-, Mordenit- oder Faujasit-Typ, insbesondere synthetische Zeolithe vom Pentasil-Typ.

Für den Begriff Pentasile gilt dabei die Definition von Kokotailo und Meier ("Pentasil family of high silicon crystalline materials" in Special Publication No. 33 of the Chemical Society, London, 1980). Die Pentasil-Familie umfaßt beispielsweise die synthetischen Zeolithe ZSM-5 (US-PS 3 702 886), ZMS-8 (GB-PS 1 334 243); ZMS-11 (US-PS 3 709 979) und ZMS-23 (US-PS 4 076 842).

Das Si/Al-Verhältnis der Pentasile liegt vorzugsweise bei 20 bis 2000, das der Mordenite bei vorzugsweise 5 bis 100. Pentasile oder Mordenite mit einem höheren Aluminiumgehalt lassen sich dabei auf das gewünschte Si/Al-Verhältnis einstellen, indem man durch Behandlung mit Mineralsäuren, organischen Säuren oder chelatisierenden Substanzen einen Teil des Aluminiums aus dem Zeolith-Gitter entfernt.

Bei dem erfindungsgemäßen Verfahren werden die Zeolithe vorzugsweise in ihrer sauren Form eingesetzt. Diese sauren Formen lassen sich nach bekannten Methoden aus den Alkalimetall-Formen, wie sie in der Regel bei der Zeolith-Synthese anfallen bzw. als Naturprodukte vorkommen, durch vollständigen oder partiellen Ionenaustausch herstellen. Eine übliche Methode zur Herstellung der H-Form eines Zeoliths besteht beispielsweise darin, die Alkalimetall-Form zunächst durch partiellen oder vollständigen Ionenaustausch mit einer Ammoniumsalz-Lösung in die Ammoniumform und diese anschließend durch Kalzinieren in die H-Form zu überführen. Aber auch die mit Alkali-, Erdalkali- und mit Seltenerdmetall-Ionen ausgetauschten Formen zeigen katalytische Aktivität.

Für das erfindungsgemäße Verfahren sind auch Zeolithe geeignet, bei denen Aluminium- oder Siliziumatome durch andere Gitteratome wie Bor, Eisen, Gallium, Germanium, Titan oder Zirkon ersetzt sind.

Die erfindungsgemäßen Zeolith-Katalysatoren bestehen im allgemeinen aus der katalytisch aktiven Zeolith-Komponente sowie einem Bindermaterial. Letzteres ist erforderlich, um den Zeolith in eine für das erfindungsgemäße Verfahren geeignete äußere Form zu bringen.

- 4 -

0175228

Als Bindermaterial eignen sich vor allem Oxide oder Hydroxide des Aluminiums und die Oxide bzw. Hydroxide des Siliciums, sowie Schichtsilicate, beispielsweise aus der Kaolin- oder Montmorillonit-Familie.

Dieser so hergestellte Zeolith-Katalysator wird gewöhnlich vor dem Einsatz in der erfindungsgemäßen Isomerisierungsreaktion zunächst durch Kalzinieren bei Temperaturen zwischen 300 und 700°C aktiviert. Zur besseren Stabilisierung des Katalysators ist es manchmal vorteilhaft, die Kalzinierung in Gegenwart von Wasserdampf, Ammoniak oder deren Mischungen durchzuführen.

Falls in der Gasphase gearbeitet werden soll, besteht eine günstige einfache Verfahrensweise zur Durchführung der erfindungsgemäßen Isomerisierung darin, daß man das Alkylanilin oder die Alkylaniline aus einer Dosiervorrichtung zuerst in eine Verdampfungszone und das entstandene Gas dann durch ein von außen beheiztes und mit dem Katalysator gefülltes Reaktionsrohr leitet. Bei Durchführung der Isomerisierung in flüssiger Phase werden das oder die Alkylaniline, ggf. verdünnt, erst erwärmt und dann in flüssiger Form durch das mit dem Katalysator gefüllte Reaktionsrohr geleitet.

In der Verdampfungs- oder Erwärmungszone erfolgt gegebenenfalls noch eine Vermischung mit Wasserstoff, Stickstoff und/oder einem anderen Trägergas, wobei Wasserstoff bevorzugt wird. Es hat sich dabei als vorteilhaft erwiesen, diese Gase vor der Vermischung auf Reaktionstemperatur aufzuheizen.

Die erfindungsgemäße Isomerisierung wird im allgemeinen bei Temperaturen zwischen 200 und 550°C, vorzugsweise bei 300 bis 450°C, sowie Drücken von 0,1 bis 10 bar, vorzugsweise bei Normaldruck, durchgeführt.

Die Belastung des Zeolith-Katalysators - ausgedrückt als LHSV (Liquid Hourly Space Velocity, $h^{-1}$) - liegt dabei im allgemeinen zwischen 0,05 und 10 $h^{-1}$, vorzugsweise zwischen 0,1 und 5 $h^{-1}$.

Die Reaktionsprodukte werden nach Verlassen des Reaktors zur Abtrennung der kondensierbaren Anteile gekühlt. Die erfindungsgemäße Isomerisierung ist jedoch nicht auf diese Verfahrensweise (Festbett-Reaktor) beschränkt, sondern läßt sich im Prinzip auch in anderen geeigneten Reaktor-Typen durchführen (z.B. Wirbelschicht-Reaktor).

Das nicht umgesetzte Ausgangsprodukt kann nach der Trennung durch Destillation, Kristallisation oder Adsorption in den katalytischen Reaktor zurückgeführt werden.

Falls die Aktivität des Katalysators langsam aufgrund einer Verkokung abnimmt, kann er von Zeit zu Zeit regeneriert werden. Dies geschieht, indem Sauerstoff, Luft, Stickstoff/Luft, Sauerstoff/Luft, Sauerstoff/Inertgas oder Luft/Inertgas bei Temperaturen zwischen 300 und 650°C über den desaktivierten Katalysator geleitet wird. Stickstoff/Luft wird dabei bevorzugt. Die Temperatur sollte dabei an keiner Stelle des Reaktors 650°C übersteigen.

Die Erfindung soll durch die folgenden Beispiele erläutert werden, wobei diese aber in keiner Weise einschränkend sein sollen.

BEISPIELE

## Katalysator-Herstellung

100 g ZSM-5 - Pulver in der Na-Form (US-PS 3 702 886, Beispiel 1) wurden bei 100°C dreimal für fünf Stunden mit 1-molarer Ammoniumchloridlösung behandelt, gewaschen, getrocknet und fünf Stunden bei 550°C in Luft calciniert. 65 g des dabei erhaltenen Pulvers wurden mit 35 g $Al_2O_3$ zu Strangpreßlingen von 1,6 mm Durchmesser verarbeitet, vier Stunden bei 500°C calciniert, auf eine Teilchengröße von 0,25 bis 1,0 mm zerkleinert und bei 450°C im Stickstoffstrom zwei Stunden lang calciniert.

## Beschreibung der Apparatur

15 ml des oben beschriebenen Katalysators wurden in einen Rohrreaktor aus Glas von 16 mm Innendurchmesser und 50 cm Länge eingefüllt und mit Glaskugeln (zum Verdampfen der flüssigen Reaktanden) überschichtet. Der Reaktor befand sich in einem elektrisch beheizten Ofen. Flüssige Reaktanden wurden über eine Dosierpumpe zugeführt, Gase über ein Gasversorgung, bestehend aus Reduzierventilen und Vorrichtungen zum Messen des Drucks und der Durchflußmenge. Die kondensierbaren Reaktionsprodukte wurden in einer Kühlfalle bei 0°C kondensiert und gaschromatographisch analysiert.

## Beispiel 1

## Isomerisierung von 2-Ethylanilin

6 ml/h 2-Ethylanilin wurden zusammen mit 1.1 l/h Wasserstoff über den oben beschriebenen Katalysator geleitet. Nach einer kurzen Vorlaufzeit von etwa 15 Minuten zur Einstellung konstanter Reaktionsbedingungen wurde stündlich die Vorlage gewechselt und anschließend analysiert. Tabelle 1 zeigt die Ergebnisse.

0175228

<u>Tabelle 1</u>: Isomerisierung von 2-Ethylanilin

| Dauer (h) | Temperatur (°C) | Anilin Gew.-% | 2-Ethylanilin (Gew.-%) | 3-Ethylanilin (Gew.-%) | 4-Ethylanilin (Gew.-%) |
|---|---|---|---|---|---|
| | | | Produktzusammensetzung | | |
| 1 | 250 | <0.1 | 99.9 | < 0.1 | < 0.1 |
| 2 | 280 | <0.1 | 99.9 | < 0.1 | < 0.1 |
| 3 | 310 | <0.1 | 99.9 | < 0.1 | < 0.1 |
| 4 | 340 | 1.2 | 86.4 | 9.3 | 2.9 |
| 5 | 370 | 4.1 | 64.3 | 25.1 | 6.3 |
| 6 | 400 | 13.5 | 39.7 | 34.2 | 8.8 |
| 7 | 430 | 27.2 | 20.7 | 37.0 | 9.6 |
| 9 | 430 | 29.4 | 17.5 | 37.8 | 10.0 |
| 13 | 430 | 27.5 | 21.1 | 39.8 | 9.9 |
| 29 | 430 | 20.8 | 27.9 | 39.6 | 10.3 |

<u>Beispiel 2</u>: (Isomerisierung 4-Ethylanilin)

Der Versuch aus Beispiel 1 wurde fortgesetzt mit 6 ml/h 4-Ethylanilin als Einsatzmaterial. Der Katalysator wurde ohne vorherige Regeneration weiterverwendet. Wie Tabelle 2 zeigt, ist die Ausbeute an 3-Ethylanilin höher als bei Verwendung von 2-Ethylanilin.

Tabelle 2 : Isomerisierung von 4-Ethylanilin

| Dauer (h) | Tempera- tur (°C) | Anilin Gew.-% | Produktzusammensetzung | | |
|---|---|---|---|---|---|
| | | | 2-Ethylanilin (Gew.-%) | 3-Ethylanilin (Gew.-%) | 4-Ethylanilin (Gew.-%) |
| 30 | 340 | 3.9 | 6.6 | 16.0 | 73.2 |
| 31 | 340 | 1.0 | 2.1 | 14.0 | 75.2 |
| 32 | 370 | 1.0 | 1.4 | 19.5 | 77.9 |
| 33 | 400 | 4.1 | 5.1 | 50.9 | 39.6 |
| 34 | 400 | 4.6 | 5.2 | 57.0 | 33.1 |
| 35 | 400 | 5.1 | 6.2 | 58.6 | 29.7 |
| 36 | 400 | 5.7 | 5.7 | 56.9 | 31.5 |
| 37 | 400 | 5.2 | 5.7 | 59.8 | 29.0 |

PATENTANSPRÜCHE:

1. Verfahren zur Isomerisierung von Alkylanilinen, deren Alkylgruppe zwei bis sechs C-Atome hat, dadurch gekennzeichnet, daß die Isomerisierung an einem Zeolith-Katalysator durchgeführt wird.

2. Verfahren nach Anspruch 1, dadurch gekennzeichnet, daß man Ethylanilin einsetzt.

3. Verfahren nach Anspruch 1 oder 2, dadurch gekennzeichnet, daß man einen Zeolith vom Pentasil-, Mordenit- oder Faujasit-Typ einsetzt.

4. Verfahren nach Anspruch 1 oder 2, dadurch gekennzeichnet, daß man einen sauren Zeolith vom Pentasil-, Mordenit- oder Faujasit-Typ einsetzt.

5. Verfahren nach Anspruch 1 oder 2, dadurch gekennzeichnet, daß man einen sauren Zeolith vom Pentasil-Typ einsetzt.

6. Verfahren nach Anspruch 4 oder 5, dadurch gekennzeichnet, daß der saure Zeolith Protonen als Kationen enthält.

7. Verfahren nach einem der Ansprüche 1 bis 6, dadurch gekennzeichnet, daß man bei Temperaturen zwischen 200°C und 550°C arbeitet.

8. Verfahren nach einem der Ansprüche 1 bis 7, dadurch gekennzeichnet, daß man bei einem Druck zwischen 0.1 bar und 10 bar arbeitet.

9. Verfahren nach einem der Ansprüche 1 bis 8, dadurch gekennzeichnet, daß man in Gegenwart von Wasserstoff, Stickstoff, Wasserdampf, Argon oder einem Gemisch aus diesen arbeitet.